# FASCICULE DE BREVET EUROPEEN

(11) **EP 1 440 674 B1**
(45) Date de publication et mention de la délivrance du brevet: **14.02.2007**
(21) Numéro de dépôt: 04300037.1
(22) Date de dépôt: 22.01.2004
(51) Int. Cl.: A61F 2/16, A61L 29/14, A61L 31/14

(54) **Cartouche pour seringue d'injection d'implant intraoculaire**
Kartusche für eine Injektionsvorrichtung einer Intraokularlinse
Cartridge for intraocular lens injection syringe

(30) Priorité: 23.01.2003 FR 0300708
(43) Date de publication de la demande: 28.07.2004
(73) Titulaire: Mehel, Eric, 44000 NANTES (FR)
(72) Inventeur: Mehel, Eric, 44000 NANTES (FR)
(74) Mandataire: Michelet, Alain

(56) Documents cités:
- WO-A-95/13022
- US-B1- 6 248 111

## Description

La présente invention concerne le domaine général de la chirurgie de l'oeil; elle concerne plus particulièrement une cartouche pour seringue d'implantation de lentilles intraoculaires (ou cristallins artificiels) au moyen d'un produit lubrifiant, par exemple de type visquo-élastique.

L'injection d'un implant dans l'oeil, par exemple lors d'une opération de la cataracte, est habituellement réalisée au moyen d'un système injecteur constitué d'une seringue et d'une cartouche d'injection, ou canule, en association avec un produit visqueux servant de lubrifiant.
Une cartouche de ce genre est notamment décrite dans le document WO-96/11649. Cette cartouche comprend une chambre de positionnement de la lentille prolongée par un tube d'injection. Lors de l'implantation, la lentille est positionnée dans la chambre de réception de la cartouche avant ou après dépose du produit lubrifiant ; cette cartouche est placée dans une seringue adaptée contenant le lubrifiant visquo-élastique et l'injection est réalisée par une manoeuvre du piston de la seringue, après insertion de l'extrémité libre du tube de la cartouche à l'intérieur de l'oeil.

Cependant, lors de l'injection de l'implant, une quantité importante de produit lubrifiant est introduite simultanément dans l'oeil, ce qui augmente la pression de l'organe opéré, entraînant un risque de lésion oculaire.

Une solution à ce problème est présentée dans le document WO-A-95/13022, qui décrit des injecteurs particuliers pour lentilles intraoculaires. En substance, les injecteurs correspondants sont pour certains équipés d'un piston dont la tête d'extrémité est munie d'orifices ou de canaux adaptés pour évacuer l'excès de produit lubrifiant à l'arrière de ladite tête, lors de la manoeuvre de ce piston pour injecter l'implant dans l'oeil.

La présente invention remédie quant à elle à ce problème en proposant un perfectionnement aux cartouches d'injection existantes, permettant de limiter la quantité de produit lubrifiant injecté dans l'oeil, sans nuire à l'efficacité de transit de la lentille dans ladite cartouche, et en particulier dans son tube d'injection.

Ainsi, la cartouche conforme à la présente invention se caractérise par le fait que la paroi de son tube d'injection comporte des moyens assurant une évacuation partielle du produit lubrifiant, en amont de l'orifice de sortie, lors de l'opération d'injection de l'implant.

Selon une caractéristique préférentielle, le tube d'injection de la cartouche comprend - une partie distale munie de l'orifice de sortie, destinée à être insérée dans l'oeil du patient lors de l'opération d'implantation, et - une partie proximale destinée à rester hors de l'oeil, laquelle partie proximale comporte les moyens assurant l'évacuation du produit lubrifiant.

Selon une forme de réalisation préférée, le tube d'injection de la cartouche est muni d'au moins un orifice pour assurer l'évacuation précitée du produit lubrifiant. De préférence les orifices correspondants sont au nombre de trois à huit, avantageusement uniformément répartis sur la partie proximale du tube d'injection.

Selon une autre particularité, le diamètre de ce ou de ces orifices est compris entre 0,01 et 2 mm, et leur forme est avantageusement cylindrique ou sensiblement cylindrique.
L'axe central de ces orifices cylindriques est de préférence incliné par rapport à l'axe central du tube d'injection, de manière à faciliter l'évacuation du produit lubrifiant lors de l'injection de l'implant. L'angle d'inclinaison correspondant est avantageusement compris entre 30 et 60°.

Selon une forme de réalisation particulière, la cartouche comporte un tube muni de six orifices cylindriques dont le diamètre est de l'ordre de 0,5 mm et dont l'inclinaison de l'axe par rapport à l'axe central de ladite cartouche est de l'ordre de 45°.

Selon une variante de réalisation possible, le tube d'injection de la cartouche est réalisé en tout ou partie en matériau poreux permettant la diffusion d'une partie du produit lubrifiant lors de l'injection de l'implant, avant son entrée dans l'oeil.

Mais l'invention sera encore illustrée, sans être aucunement limitée, par la description suivante d'un mode de réalisation particulier, donné uniquement à titre d'exemple et représenté sur les dessins annexés dans lesquels :
- la figure 1 est une vue en perspective d'une cartouche pour seringue d'injection d'implant oculaire conforme à l'invention ;
- la figure 2 est une vue en coupe longitudinale de la cartouche représentée sur la figure 1 ;
- la figure 3 est une vue partielle agrandie du tube d'injection de la cartouche illustrée figure 2.

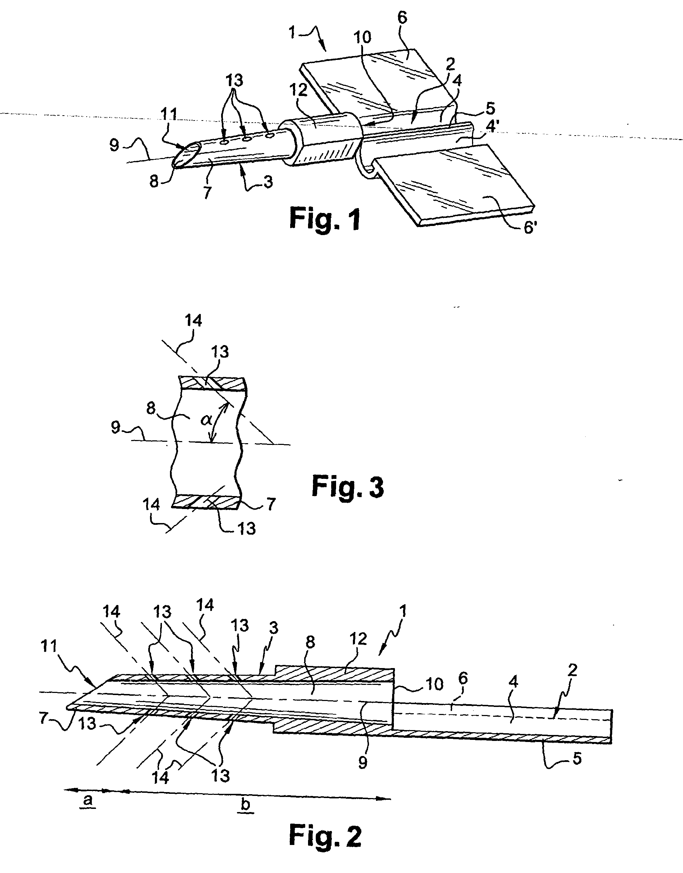

La cartouche 1 représentée sur les figures 1 à 3 est du type de celle décrite dans le document WO-96/11649. Cette cartouche est destinée à recevoir un implant intraoculaire, non représenté, pour assurer son injection dans l'oeil d'un patient au moyen d'une seringue appropriée, et ceci par l'intermédiaire d'un produit lubrifiant, par exemple un produit visquo-élastique, destiné à faciliter son trajet.

Le document WO-95/13022 divulgue une cartouche pour seringue d'injection d'implant intraoculaire en association avec un produit lubrifiant selon le préambule de la revendication 1.

De manière classique, la cartouche 1 comprend une chambre 2 d'accueil de l'implant, prolongée par un tube d'injection 3. La chambre 2 est constituée de deux demi-tubes 4, 4' dont l'un est mobile, reliés par une ligne de liaison 5 formant charnière. Les deux demi-tubes 4, 4' comportent chacun au niveau de leur bordure d'extrémité libre, opposée à la charnière 5, un volet plan 6, 6' de forme générale rectangulaire constituant des organes de préhension pour les manoeuvres d'ouverture et de fermeture de la chambre 2.

Sur les figures 1 et 2, la chambre 2 est représentée dans une configuration ouverte. Sa position fermée est obtenue en faisant pivoter le demi-tube mobile 4' autour de la charnière 5 de manière à venir plaquer l'un contre l'autre les deux volets 6, 6'.
Une fois réunies, les deux structures demi-cylindriques 4, 4' forment une chambre tubulaire 2.

Le tube d'injection 3 est réalisé monobloc avec les éléments 4, 4', 5, 6, 6' formant la chambre d'accueil 2.
Ce tube d'injection 3 comporte une paroi 7 qui définit un canal interne 8, d'axe central 9, délimité par un orifice d'entrée 10 permettant sa connexion à la chambre 2, et par un orifice de sortie 11 au niveau de son extrémité libre. L'orifice de sortie 11 est biseauté pour faciliter l'insertion du tube 3 dans l'incision de l'oeil, en vue de l'implantation de la lentille.
Le diamètre interne du canal 8 se réduit légèrement depuis l'orifice d'entrée 10 jusqu'à l'orifice de sortie 11 ; du côté de la chambre d'accueil 2, on remarque que le tube d'injection 3 comporte une épaisseur de paroi plus importante constituant une zone de renforcement 12.

Le tube d'injection 3 comprend une partie d'extrémité a, dite « partie distale » qui est destinée à venir s'insérer dans l'oeil au moment de l'opération d'injection ; cette partie distale a s'étend généralement sur 3 à 4 mm. Le reste b de la longueur du tube 3 est destiné à rester hors de l'oeil lors de l'opération d'injection. Cette partie externe b est appelée « partie proximale » ; elle s'étend depuis la partie distale a jusqu'à l'orifice d'entrée 10.

La cartouche 1 conforme à l'invention comporte des moyens qui, au cours de l'opération d'injection de l'implant, permettent d'assurer une évacuation partielle du produit lubrifiant accompagnant l'implant, en amont de l'orifice de sortie 11 du tube d'injection 3, et en particulier en amont de la partie distale a.

Tels que représentés, ces moyens d'évacuation du produit lubrifiant consistent ici en une pluralité d'orifices 13 aménagés dans la partie proximale b du tube d'injection 3, et en particulier sur la zone située entre la partie distale a et la structure de renforcement 12.

Dans l'exemple de réalisation illustré, ces orifices 13 sont au nombre de six, répartis par couples diamétralement opposés. Ces orifices 13 ont une forme générale cylindrique d'axe central 14 ; leur diamètre est avantageusement compris entre 0,01 et 2 mm, de préférence de l'ordre de 0,5 mm.

En outre, l'axe 14 des orifices cylindriques 13 est incliné vers l'avant, c'est-à-dire vers l'orifice de sortie 11, pour faciliter l'évacuation du produit lubrifiant lors de l'opération d'injection de l'implant. L'axe correspondant 14 forme avantageusement un angle aigu avec l'axe central 9 du tube d'injection 3, compris entre 30 et 60°, de préférence voisin de 45°.

Les orifices 13 aménagés dans le tube d'injection 3 peuvent être réalisés lors du moulage de la cartouche, ou postérieurement par une opération appropriée de poinçonnage ou de perçage. Cette opération de poinçonnage ou de perçage peut être réalisée manuellement ou de manière automatisée.

Bien entendu, il est possible d'agir sur les différents paramètres, à savoir le nombre, la taille, la forme et la position des orifices, pour assurer une évacuation optimale du produit lubrifiant de la cartouche, tout en maintenant une quantité suffisante de produit pour ne pas nuire à la circulation de l'implant.

L'association d'orifices 13 peut par exemple permettre d'évacuer les 2/3 du produit lubrifiant ; seul le tiers restant sera injecté au sein de l'oeil simultanément à l'insertion de la lentille intraoculaire ;

En pratique, la chambre 2 de la cartouche, en position ouverte, permet la réception de l'implant avec le produit lubrifiant. Cette chambre 2 est fermée au moyen des volets plans 6 qui viennent se plaquer l'un contre l'autre.
La cartouche 1 peut alors être positionnée sur sa seringue d'accueil pour assurer l'opération d'implantation. Le praticien actionne le piston de la seringue qui pousse l'implant avec le produit lubrifiant ; l'implant se déplace dans le canal tubulaire 8 du tube 3 et sort au sein de l'oeil opéré par l'orifice de sortie 11 ; au cours du transit de l'implant dans le tube 3, une partie du produit lubrifiant est évacuée par l'intermédiaire des orifices 13 de manière à limiter la quantité injectée dans l'oeil.

L'invention permet d'éviter la pénétration d'une quantité trop importante de produit lubrifiant dans l'oeil ; on réduit ainsi les risques de lésions oculaires, en particulier dans le domaine de la chirurgie de la cataracte.

Dans une variante de réalisation, non représentée, le tube d'injection de la cartouche peut être réalisé en tout ou partie en matériau poreux permettant une diffusion du produit lubrifiant au moment de l'injection de l'implant.

Bien entendu, la présente invention ne s'applique pas uniquement au modèle de cartouche présenté sur les figures ; on peut de la même manière envisager son application sur tout type de cartouche utilisant un produit lubrifiant accompagnant le déplacement de l'implant.

## Revendications

1. Cartouche pour seringue d'injection d'implant intraoculaire en association avec un produit lubrifiant, laquelle cartouche (1) comprend une chambre (2) de réception de l'implant prolongée par un tube d'injection (3) muni d'un orifice d'extrémité (11) permettant la sortie dudit implant accompagné du produit lubrifiant, **caractérisée en ce qu'**elle comporte des moyens (13) aménagés dans la paroi (7) du tube d'injection (3), assurant une évacuation partielle dudit produit lubrifiant en amont de l'orifice de sortie (11) du tube d'injection (3), au cours de l'opération d'injection de l'implant.

2. Cartouche selon la revendication 1, **caractérisée en ce que** le tube d'injection (3) comprend une partie distale (a) munie de l'orifice de sortie (11), destinée à être insérée dans l'oeil du patient lors de l'opération d'implantation, et une partie proximale (b) destinée à rester hors de l'oeil, laquelle partie proximale (b) comporte les moyens (13) assurant l'évacuation du produit lubrifiant.

3. Cartouche selon l'une quelconque des revendications 1 ou 2, **caractérisée en ce que** les moyens assurant l'évacuation partielle du produit lubrifiant consistent en au moins un orifice (13) aménagé dans la paroi (7) du tube d'injection (3).

4. Cartouche selon la revendication 3, **caractérisée en ce que** le tube d'injection (3) comporte une pluralité d'orifices (13) répartis uniformément sur la paroi (7) de sa partie proximale (b).

5. Cartouche selon l'une quelconque des revendications 3 ou 4, **caractérisée en ce que** le tube d'injection (3) comporte trois à huit orifices (13).

6. Cartouche selon l'une quelconque des revendications 3 à 5, **caractérisée en ce que** le tube d'injection (3) comporte un ou des orifices (13) de forme cylindrique ou sensiblement cylindrique.

7. Cartouche selon la revendication 6, **caractérisée en ce que** le tube d'injection (3) comporte un ou des orifices (13) dont le diamètre est compris entre 0,01 et 2 mm.

8. Cartouche selon l'une quelconque des revendications 6 ou 7, **caractérisée en ce que** le tube d'injection (3) comporte un ou des orifices (13) dont l'axe central (14) est incliné par rapport à l'axe central (9) dudit tube (3), de manière à faciliter l'évacuation du produit lubrifiant lors de l'injection de l'implant.

9. Cartouche selon la revendication 8, **caractérisée en ce que** l'angle d'inclinaison α de l'axe (14) des orifices (13) par rapport à l'axe central (9) du tube d'injection (3) est compris entre 30 et 60°.

10. Cartouche selon l'une quelconque des revendications 6 à 9, **caractérisée en ce qu**'elle comporte un tube d'injection (3) muni de six orifices cylindriques (13) dont le diamètre est de l'ordre de 0,5 mm, et dont l'inclinaison α de l'axe (14) par rapport à l'axe central (9) dudit tube (3) est de l'ordre de 45°.

11. Cartouche selon l'une quelconque des revendications 1 ou 2, **caractérisée en ce qu**'elle comporte un tube d'injection constitué en tout ou partie en matériau poreux assurant une évacuation partielle du produit lubrifiant.

## Claims

1. A cartridge for intraocular lens injection syringe in combination with a lubricating product, said cartridge (1) including a chamber (2) for accommodating the lens extended by an injection tube (3) fitted with an end orifice (11) enabling extraction of said lens accompanied by the lubricating product; **characterised in that** it comprises means (13) provided in the wall (7) of the injection tube (3), ensuring partial evacuation of said lubricating product upstream of the outlet orifice (11) of the injection tube (3), during the lens injection operation.

2. A cartridge according to claim 1, **characterised in that** the injection tube (3) includes a distal portion (a) fitted with the outlet orifice (11), intended for being inserted into the eye of the patient during the implantation operation, and a proximal portion (b) intended for remaining outside the eye, which proximal portion (b) includes the means (13) ensuring the evacuation of the lubricating product.

3. A cartridge according to any of the claims 1 or 2, **characterised in that** the means ensuring partial evacuation of the lubricating product consist of at least one orifice (13) provided in the wall (7) of the injection tube (3).

4. A cartridge according to claim 3, **characterised in that** the injection tube (3) comprises a plurality of orifices (13) evenly distributed over the wall (7) of its proximal portion (b).

5. A cartridge according to any of the claims 3 or 4, **characterised in that** the injection tube (3) includes three to eight orifices (13).

6. A cartridge according to any of the claims 3 to 5, **characterised in that** the injection tube (3) includes one or several orifices (13) of cylindrical or substantially cylindrical shape.

7. A cartridge according to claim 6, **characterised in that** the injection tube (3) includes one or several orifices (13) whereof the diameter ranges between 0.01 and 2 mm.

8. A cartridge according to any of the claims 6 or 7, **characterised in that** the injection tube (3) comprises one or several orifices (13) whereof the central axis (14) is tilted with respect to the central axis (9) of said tube (3), in order to facilitate the evacuation of the lubricating product when injecting the lens.

9. A cartridge according to claim 8, **characterised in that** the tilting angle a of the axis (14) of the orifices (13) relative to the central axis (9) of the injection tube (3) ranges between 30 and 60°.

10. A cartridge according to any of the claims 6 to 9, **characterised in that** it comprises an injection tube (3) fitted with six cylindrical orifices (13) whereof the diameter is of the order of 0.5 mm and whereof the inclination a of the axis (14) relative to the central axis (9) of said tube (3) is of the order of 45°.

11. A cartridge according to any of the claims 1 or 2, **characterised in that** it comprises an injection tube formed totally or partially of a porous material ensuring partial evacuation of the lubricating product.

## Patentansprüche

1. Kartusche für eine Spritze zum Injizieren eine intraokularen Implantats in Verbindung mit einem Gleitmittel, wobei die Kartusche (1) eine Kammer (2) zum Aufnehmen des Implantats aufweist, welche durch ein Injektionsröhrchen (3) verlängert ist, das mit einer Endöffnung (11) versehen ist, welche das Austreten des Implantats zusammen mit dem Gleitmittel ermöglicht; **dadurch gekennzeichnet, dass** sie in der Wand (7) des Injektionsröhrchens (3) ausgebildete Einrichtungen (13) aufweist, welche im Verlauf des Injizierend des Implantats ein teilweises Auslassen des Gleitmittels stromaufwärts des Austrittsöffnung (11) des Injektionsröhrchens (3) ermöglichen.

2. Kartusche nach Anspruch 1, **dadurch gekennzeichnet, dass** das Injektionsröhrchen (3) einen mit der Austrittsöffnung (11) versehenen distalen Teil (a), der zum Einführen in das Auge eines Patienten während des Implantierens vorgesehen Ist, und einen proximalen Teil (b), der außerhalb des Auges verbleibt, wobei der proximale Teil (b) die das Auslassen des Gleitmittels gewährleistenden Einrichtungen (13) aufweist.

3. Kartusche nach einem der Ansprüche 1 oder 2, **dadurch gekennzeichnet, dass** die das teilweise Auslassen des Gleitmittels gewährleistenden Einrichtungen (13) aus mindestens einer Öffnung (13) bestehen, die in der Wand (7) des Injektionsröhrchens (3) ausgebildet ist.

4. Kartusche nach Anspruch 3, **dadurch gekennzeichnet, dass** das Injektionsröhrchen (3) mehrere Öffnungen (13) aufweist, welche gleichmäßig über die Wand (7) seines proximalen Teils (b) verteilt sind.

5. Kartusche nach einem der Ansprüche 3 oder 4, **dadurch gekennzeichnet, dass** das Injektionsröhrchen (3) drei bis acht Öffnungen (13) aufweist.

6. Kartusche nach einem der Ansprüche 3 bis 5, **dadurch gekennzeichnet, dass** das Injektionsröhrchen (3) eine Öffnung oder Öffnungen (13) mit zylindrischer oder im wesentlichen zylindrischer Form aufweist.

7. Kartusche nach Anspruch 6, **dadurch gekennzeichnet, dass** das Injektionsröhrchen (3) eine Öffnung oder Öffnungen (13) aufweist, deren Durchmesser zwischen 0,01 und 2 mm liegt.

8. Kartusche nach einem der Ansprüche 6 oder 7, **dadurch gekennzeichnet, dass** das Injektionsröhrchen (3) eine Öffnung oder Öffnungen (13) aufweist, deren Mittelachse (14) in bezug auf die Mittelachse (9) des Röhrchens (3) derart geneigt ist, dass das Auslassen des Gleitmittels beim Injizieren des Implantats vereinfacht ist.

9. Kartusche nach Anspruch 8, **dadurch gekennzeichnet, dass** der Neigungswinkel α der Achse (14) der Öffnungen (13) in bezug auf die Mittelachse (9) des Injektionsröhrchens (3) zwischen 30 und 60° liegt.

10. Kartusche nach einem der Ansprüche 6 bis 9, **dadurch gekennzeichnet, dass** sie ein Injektionsröhrchen (3) aufweist, das mit zylindrischen Öffnungen (13) versehen ist, deren Durchmesser in der Größenordnung von 0,5 mm liegt und deren Neigung α der Achse (14) in bezug auf die Mittelachse (9) des Röhrchens (3) in der Größenordnung von 45° liegt.

11. Kartusche nach einem der Ansprüche 1 oder 2, **dadurch gekennzeichnet, dass** sie ein Injektionsröhrchen aufweist, das vollständig oder teilweise aus porösem Material besteht, das ein teilweises Auslassen des Gleitmittels ermöglicht.
